# EUROPEAN PATENT APPLICATION

(11) **EP 3 838 420 A1**
(43) Date of publication of application: **23.06.2021**
(21) Application number: 20167015.5
(22) Date of filing: 31.03.2020
(51) Int. Cl.: B05B 17/06, B05B 1/00, A61L 9/14, F24F 6/00

(54) **ADAPTIVE WATER-LEVEL NOISE-REDUCING ATOMIZING DEVICE**

(30) Priority: 20.12.2019 CN 201922312629 U
(71) Applicant: Shenzhen Dituo Electronic Co., Ltd., Shenzen, Guangdong 518125 (CN)
(72) Inventor: HU, Yangmin, Shenzen, Guangdong 518125 (CN); TANG, Xiaolan, Shenzen, Guangdong 518125 (CN); ZHANG, Zuoyun, Shenzen, Guangdong 518125 (CN)
(74) Representative: Sebastian, Jens

(57) **Abstract**

Disclosed is an adaptive water-level noise-reducing atomizing device, comprising a bottom shell, an upper shell cover positioned on the bottom shell, a water storage tank arranged on a top surface of the bottom shell, a mist outlet arranged at a top end of the upper shell cover, a coupling sleeve fixed on an inner wall surface of the upper shell cover, a silent hood sleeved on the coupling sleeve and being extendable to the bottom of the water storage tank, and a wave collecting ring having lower density than liquid in the tank arranged in the silent hood and capable of moving up and down. An ultrasonic atomizer protruding into the water storage tank is fixed in the middle of the bottom shell, a top end of the silent hood is sleeved with the coupling sleeve and covers the periphery of the ultrasonic atomizer.

## Description

### TECHNICAL FIELD

The present application discloses an adaptive water-level noise-reducing atomizing device.

### BACKGROUND

Ultrasonic atomizing type aromatherapy machines and humidifiers are such devices in which ultrasonic atomizing sheets are used to generate high-frequency mechanical waves exciting water and essential oils to cause their molecules or molecular groups to escape from the liquid surface and float in the form of a mist on the liquid surface, and then the molecules or molecular groups are blown by an air supply system into the ambient air to complete the atomizing operation.

In practical applications, the ultrasonic atomizing sheet has an optimal operating depth. That is to say, at the optimal operating depth, the ultrasonic atomizing sheet is in the best operating state with the highest energy efficiency (atomizing amount per unit of electrical energy) and the longest life of the atomizing sheet.

The optimal operating depth of the ultrasonic atomizing sheet limits the capacity and shape of the water storage tank, making it difficult to meet the needs of large-capacity humidifiers.

### SUMMARY

The present application aims at the above-mentioned shortcomings of the prior art, and provides an adaptive water-level noise-reducing atomizing device.

The technical solution adopted by the present application to solve its technical problems is to construct an adaptive water-level noise-reducing atomizing device, comprising a bottom shell, an upper shell cover positioned on the bottom shell, a water storage tank arranged on a top surface of the bottom shell, a mist outlet arranged at a top end of the upper shell cover, a coupling sleeve fixed on an inner wall surface of the upper shell cover, a silent hood sleeved on the coupling sleeve and being extendable to the bottom of the water storage tank, and a wave collecting ring arranged in the silent hood in a manner capable of moving up and down, wherein the density of the wave collecting ring is less than or equal to that of liquid in the water storage tank, an ultrasonic atomizer protruding into the water storage tank is fixed in the middle of the bottom shell, a top end of the silent hood is sleeved with the coupling sleeve, and the top end of the silent hood is positioned on and covers the periphery of the ultrasonic atomizer.

In some embodiments of the adaptive water-level noise-reducing atomizing device according to the present application, a water baffle plate may be arranged in the coupling sleeve at a position directly above the ultrasonic atomizer.

In some embodiments of the adaptive water-level noise-reducing atomizing device according to the present application, a first connection seat may be fixed on the inner wall surface of the upper shell cover and at a first side of the mist outlet, a bottom end of the first connection seat being provided with a first connection hole, a second connection seat may be fixed on the inner wall surface of the upper shell cover and at a second side of the mist outlet, a bottom end of the second connection seat being provided with a second connection hole, a first side of a top surface of the water baffle plate may be provided with a first insertion post which can be inserted into the first connection hole for connection, and a second side of the top surface of the water baffle plate may be provided with a second insertion post which can be inserted into the second connection hole for connection.

In some embodiments of the adaptive water-level noise-reducing atomizing device according to the present application, the water baffle plate may be a circular water baffle plate with a convex middle and a low peripheral edge.

In some embodiments of the adaptive water-level noise-reducing atomizing device according to the present application, the silent hood may comprise a beam wave tube, an atomizing cavity tube arranged at the top of the beam wave tube, and a droplet receiving tube connecting the beam wave tube and the atomizing cavity tube, the droplet receiving tube being a conical tube, the droplet receiving tube being located directly below a peripheral edge of the water baffle plate, and a top end of the atomizing cavity tube being sleeved with the coupling sleeve.

In some embodiments of the adaptive water-level noise-reducing atomizing device according to the present application, a plurality of first air inlet holes may be arranged at a connection between the droplet receiving tube and the atomizing cavity tube, and the beam wave tube may be provided with a water inlet hole through which water in the water storage tank enters the beam wave tube.

In some embodiments of the adaptive water-level noise-reducing atomizing device according to the present application, a side wall of the atomizing cavity tube may be provided with an air guide hood which is in communication with the atomizing cavity tube and which is used for guiding the mist out of the atomizing cavity tube from the side of the atomizing cavity tube.

In some embodiments of the adaptive water-level noise-reducing atomizing device according to the present application, the wave collecting ring may comprise a main body and a wave passage hole provided in a middle portion of the main body and penetrating the main body vertically.

In some embodiments of the adaptive water-level noise-reducing atomizing device according to the present application, the wave collecting ring may be made of PP plastic material, and support ribs are arranged on an outer side wall and / or a bottom wall of the main body.

In some embodiments of the adaptive water-level noise-reducing atomizing device according to the present application, the bottom shell may be provided with an air inlet fan that blows air into the upper shell cover, an inner wall surface of the upper shell cover may be provided with a sealing hood positioned on a top end of the water storage tank to sealably cover it, and the sealing hood or the water storage tank may be provided with a second air inlet hole allowing external air to be blown into the water storage tank.

The implementation of embodiments of adaptive water-level noise-reducing atomizing device according to the present application has the following beneficial effects. When using the adaptive water-level noise-reducing atomizing device according to the embodiments of the present application, after power is applied, the ultrasonic atomizer located at the bottom of the water storage tank generates high-frequency mechanical waves. Because the density of the wave collecting ring is smaller than the density of the liquid in the water storage tank, the wave collecting ring is located in the silent hood and partially floats above the liquid level in the water storage tank, and further the silent hood is located on the periphery of the ultrasonic atomizer, following effect can be achieved. That is, in the process of generating high-frequency mechanical waves by the ultrasonic atomizer, the silent hood and the wave collecting ring can reduce the transmission of high-frequency mechanical waves to the surroundings, so that the high-frequency mechanical waves generated by the ultrasonic atomizer can be transmitted upward for a longer distance, thereby playing the role of wave collection. The high-frequency mechanical waves excite the liquid column in the silent hood and the wave collecting ring, and a mist above the liquid level in the wave collecting ring is formed. The mist enters the top end of the silent hood and is discharged from the mist outlet to achieve the atomization effect. Thus, the operating liquid level of the ultrasonic atomizer is increased, and the operating efficiency of the ultrasonic atomizer is improved.

### BRIEF DESCRIPTION OF THE DRAWINGS

The present application will be further described below with reference to the accompanying figures and embodiments. In the figures:
Fig. 1 is a schematic sectional structure diagram of a adaptive water-level noise-reducing atomizing device according to some embodiments of the present application;
Fig. 2 is a schematic structural diagram of a silent hood in the adaptive water-level noise-reducing atomizing device according to some embodiments of the present application;
Fig. 3 is schematic structural diagram of the silent hood in the adaptive water-level noise-reducing atomizing device according to other embodiments of the present application;
Fig. 4 is schematic structure diagram of the adaptive water-level noise-reducing atomizing device according to other embodiments of the present application;
Fig. 5 is a schematic structural diagram of a wave collecting ring in the adaptive water-level noise-reducing atomizing device according to some embodiments of the present application; and
Fig. 6 is a schematic structural diagram of the wave collecting ring in the adaptive water-level noise-reducing atomizing device according to some embodiments of the present application.

### DETAILED DESCRIPTION

In order to make the purpose, technical solution and advantages of the present application clear, some embodiments of the present application will be further described in detail below with reference to the accompanying figures.

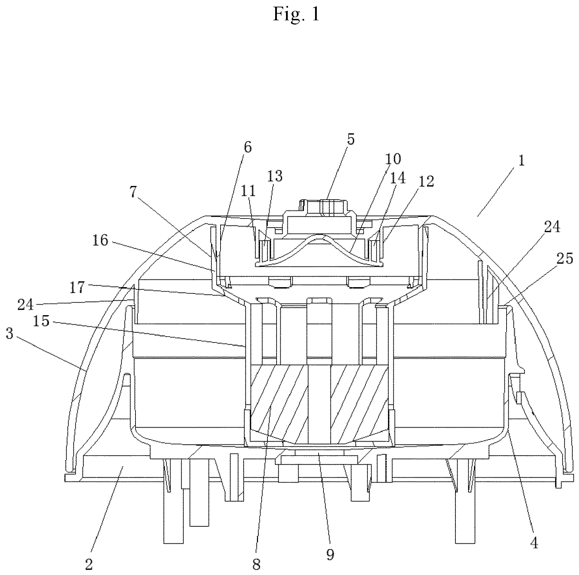

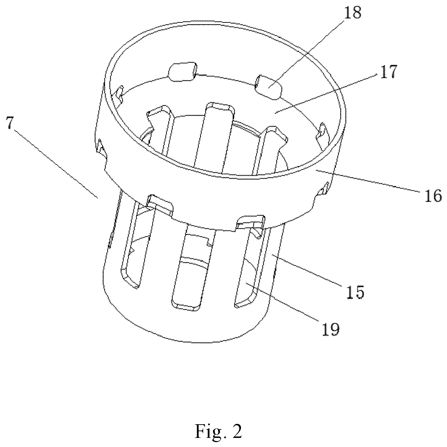

As shown in Figs. 1, 2, and 5, in a first embodiment of the adaptive water-level noise-reducing atomizing device according to the present application, the atomizing device 1 comprises a bottom shell 2, an upper shell cover 3 positioned on the bottom shell 2, a water storage tank 4 arranged on a top surface of the bottom shell 2, a mist outlet 5 arranged at a top end of the upper shell cover 3, a coupling sleeve 6 fixed on an inner wall surface of the upper shell cover 3, a silent hood 7 sleeved on the coupling sleeve 6 and being extendable to the bottom of the water storage tank 4, and a wave collecting ring 8 arranged in the silent hood 7 in a manner capable of moving up and down, wherein the density of the wave collecting ring 8 is less than or equal to that of a liquid in the water storage tank 4, an ultrasonic atomizer 9 protruding into the water storage tank 4 is fixed in the middle of the bottom shell 2. A top end of the silent hood 7 is sleeved with the coupling sleeve 6 and is positioned on and covers the periphery of the ultrasonic atomizer 9.

When using the adaptive water-level noise-reducing atomizing device 1 according to the embodiments of the present application, after power is applied, the ultrasonic atomizer 9 located at the bottom of the water storage tank 4 generates high-frequency mechanical waves. Because the density of the wave collecting ring 8 is smaller than the density of the liquid in the water storage tank 4, the wave collecting ring 8 is located in the silent hood 7 and partially floats above the liquid level in the water storage tank 4, and further the silent hood 7 is located on the periphery of the ultrasonic atomizer 9, following effect can be achieved. That is, in the process of generating high-frequency mechanical waves by the ultrasonic atomizer 9, the silent hood 7 and the wave collecting ring 8 can reduce the transmission of high-frequency mechanical waves to the surroundings, so that the high-frequency mechanical waves generated by the ultrasonic atomizer 9 can be transmitted upward for a longer distance, thereby playing the role of wave collection. The high-frequency mechanical waves excite the liquid column in the silent hood 7 and the wave collecting ring 8, and mist above the liquid level in the wave collecting ring 8 is formed. The mist enters the top end of the silent hood 7 and is discharged from the mist outlet 5 to achieve the atomization effect. Thus, the operating liquid level of the ultrasonic atomizer 9 is increased, and the operating efficiency of the ultrasonic atomizer 9 is improved.

Preferably, in this embodiment, the mist outlet 5 is disposed at the middle of the top end of the upper shell cover 3.

Further, in order to prevent liquid from splashing upward upon being excited by the ultrasonic atomizer 9, a water baffle plate 10 is arranged in the coupling sleeve 6 at a position directly above the ultrasonic atomizer 9. The water baffle plate 10 can baffle water droplets splashing upward.

Specifically, a first connection seat 11 is fixed on the inner wall surface of the upper shell cover 3 on a first side of the mist outlet 5, a bottom end of the first connection seat 11 is provided with a first connection hole, a second connection seat 12 is fixed on the inner wall surface of the upper shell cover 3 on a second side of the mist outlet 5, a bottom end of the second connection seat 12 is provided with a second connection hole, a first side of a top surface of the water baffle plate 10 is provided with a first insertion post 13 which can be inserted into the first connection hole for connection, and a second side of the top surface of the water baffle plate 10 is provided with a second insertion post 14 which can be inserted into the second connection hole for connection. The water baffle plate 10 can be formed as a circular water baffle plate 10 with a convex middle and a low peripheral edge, for example in the form of a bamboo hat or streamlined hat.

In the present embodiment, the silent hood 7 comprises a beam wave tube 15, an atomizing cavity tube 16 arranged at a top end of the beam wave tube 15, and a droplet receiving tube 17 connecting the beam wave tube 15 and the atomizing cavity tube 16. The droplet receiving tube 17 is a conical tube which is located directly below a peripheral edge of the water baffle plate 10. A top end of the atomizing cavity tube 16 is sleeved with the coupling sleeve 6. During the atomization process, the mist or water droplets intercepted by the water baffle plate 10 can be dripped onto the droplet receiving tube 17 along the periphery of the water baffle plate 10 and returns to the water storage tank 4 to prevent the water droplets from directly dripping on the liquid surface to thus generate dripping sound, thereby achieving the noise reduction effect.

Further, a plurality of first air inlet holes 18 are arranged at a connection between the droplet receiving tube 17 and the atomizing cavity tube 16, and the beam wave tube 15 is provided with a water inlet hole 19 through which water in the water storage tank 4 enters the beam wave tube to permit atomization by the ultrasonic atomizer 9.

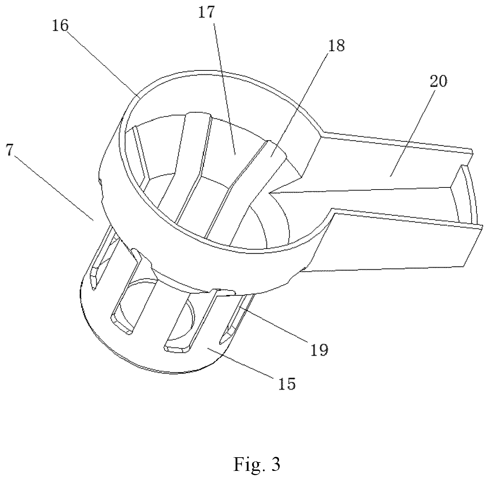

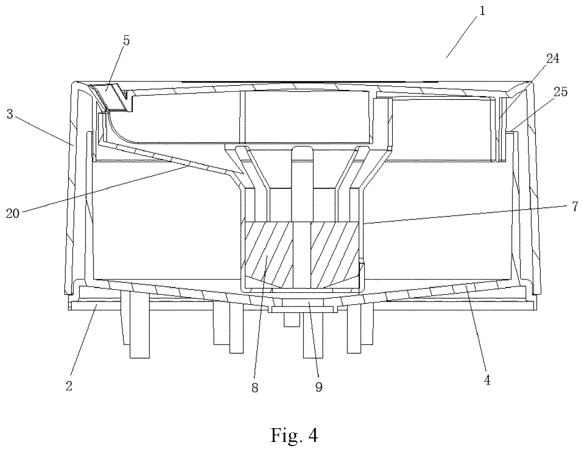

In another embodiment, as shown in Figs. 3 and 4, in order to facilitate the guiding of the mist from the side of the atomizing device 1, a side wall of the atomizing cavity tube 16 is provided with an air guide hood 20 which is in communication with the atomizing cavity tube 16 and is used for guiding the mist out of the atomizing cavity tube 16 from the side of the atomizing cavity tube 16. In the present embodiment, the mist outlet 5 is provided at a side of the top end of the upper shell cover 3.

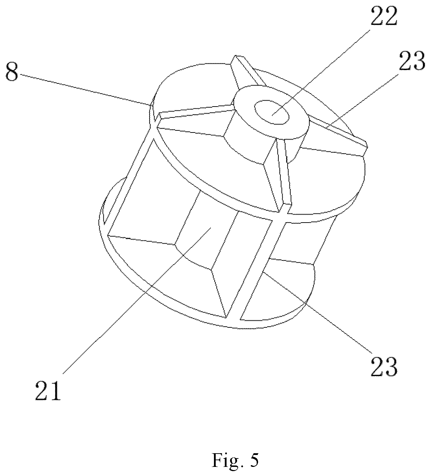

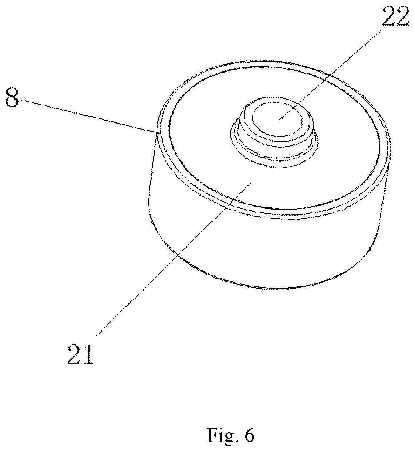

In this embodiment, as shown in Fig. 5 or 6, the wave collecting ring 8 comprises a main body 21 and a wave passage hole 22 provided in a middle portion of the main body 21 and penetrating the main body 21 vertically. During the atomization process, the high-frequency mechanical waves generated by the ultrasonic atomizer 9 are transmitted upward into the wave passage hole 22, and liquid in the wave passage hole 22 is atomized. Thus, the upward transmission distance of the high-frequency mechanical waves can be increased and the better wave collecting effect can be achieved.

Further, as shown in Fig. 4, in order to facilitate removal of the wave collecting ring 8 from the beam wave tube 15, the wave collecting ring 8 is made of PP plastic material, and support ribs 23 are arranged on an outer side wall and / or a bottom wall of the main body 21.

In this embodiment, the bottom shell 2 is provided with an air inlet fan (not shown) that blows air into the upper shell cover 3, an inner wall surface of the upper shell cover 3 is provided with a sealing hood 24 which is positioned on a top end of the water storage tank 4 to sealably covers it, and the sealing hood 24 or the water storage tank 4 is provided with a second air inlet hole 25 allowing external air to be blown into the water storage tank 4.

During the atomization process, the air inlet fan blows external air into the atomizing device 1, and the external air is blown into the water storage tank 4 through the second air inlet hole 25, and then enters the silent hood 7 through the first air inlet hole 18. The mist generated by atomization in the silent hood 7 is discharged along with the air to the outside of the atomizing device 1 through the mist outlet 5, thereby achieving atomization and air circulation.

The above description is only embodiments of the present application, and hence does not limit the scope of the patent of the present application. Any equivalent structure or equivalent process transformation made by using the specification and drawings of the application, or directly or indirectly used in other related technical fields, is also included in the scope of patent protection of the application.

## Claims

1. An adaptive water-level noise-reducing atomizing device, comprising a bottom shell,
an upper shell cover positioned on the bottom shell,
a water storage tank arranged on a top surface of the bottom shell,
a mist outlet arranged at a top end of the upper shell cover,
a coupling sleeve fixed on an inner wall surface of the upper shell cover,
a silent hood sleeved on the coupling sleeve and being extendable to the bottom of the water storage tank, and
a wave collecting ring arranged in the silent hood in a manner capable of moving up and down,
wherein the density of the wave collecting ring is less than or equal to that of liquid in the water storage tank, an ultrasonic atomizer protruding into the water storage tank is fixed in the middle of the bottom shell, and a top end of the silent hood is sleeved with the coupling sleeve and covers the periphery of the ultrasonic atomizer.

2. The adaptive water-level noise-reducing atomizing device according to claim 1, wherein a water baffle plate is arranged in the coupling sleeve at a position directly above the ultrasonic atomizer.

3. The adaptive water-level noise-reducing atomizing device according to claim 2, wherein a first connection seat is fixed on the inner wall surface of the upper shell cover and at a first side of the mist outlet, a bottom end of the first connection seat is provided with a first connection hole, a second connection seat is fixed on the inner wall surface of the upper shell cover and at a second side of the mist outlet, a bottom end of the second connection seat is provided with a second connection hole, a first side of a top surface of the water baffle plate is provided with a first insertion post which can be inserted into the first connection hole for connection, and a second side of the top surface of the water baffle plate is provided with a second insertion post which can be inserted into the second connection hole for connection.

4. The adaptive water-level noise-reducing atomizing device according to claim 3, wherein the water baffle plate is a circular water baffle plate with a convex middle and a lower peripheral edge.

5. The adaptive water-level noise-reducing atomizing device according to claim 4, wherein the silent hood comprises a beam wave tube, an atomizing cavity tube arranged at a top end of the beam wave tube, and a droplet receiving tube connecting the beam wave tube and the atomizing cavity tube, the droplet receiving tube is a conical tube, the droplet receiving tube is located directly below a peripheral edge of the water baffle plate, and a top end of the atomizing cavity tube is sleeved with the coupling sleeve.

6. The adaptive water-level noise-reducing atomizing device according to claim 5, wherein a plurality of first air inlet holes are arranged at a connection between the droplet receiving tube and the atomizing cavity tube, and the beam wave tube is provided with a water inlet hole through which water in the water storage tank enters the beam wave tube.

7. The adaptive water-level noise-reducing atomizing device according to claim 6, wherein a side wall of the atomizing cavity tube is provided with an air guide hood which is in communication with the atomizing cavity tube and which is used for guiding the mist out of the atomizing cavity tube from the side of the atomizing cavity tube.

8. The adaptive water-level noise-reducing atomizing device according to claim 6, wherein the wave collecting ring comprises a main body and a wave passage hole provided in a middle portion of the main body and penetrating the main body vertically.

9. The adaptive water-level noise-reducing atomizing device according to claim 8, wherein the wave collecting ring is made of PP plastic material, and support ribs are arranged on an outer side wall and / or a bottom wall of the main body.

10. The adaptive water-level noise-reducing atomizing device according to claim 1, wherein the bottom shell is provided with an air inlet fan that blows air into the upper shell cover, an inner wall surface of the upper shell cover is provided with a sealing hood positioned on and sealably covers a top end of the water storage tank, and the sealing hood or the water storage tank is provided with a second air inlet hole allowing external air to be blown into the water storage tank.
